# EUROPÄISCHE PATENTSCHRIFT

(11) **EP 2 309 941 B1**
(45) Veröffentlichungstag und Bekanntmachung des Hinweises auf die Patenterteilung: **24.10.2018**
(21) Anmeldenummer: 09776812.1
(22) Anmeldetag: 23.06.2009
(51) Int. Cl.: A61B 18/14, A61B 18/02

(54) **ELEKTROCHIRURGISCHES GERÄT ZUR ERZEUGUNG EINER VORGEGEBENEN WÄRMEVERTEILUNG ÜBER EINEN SONDENKÖRPER**
ELECTROSURGICAL DEVICE FOR GENERATING A PRESCRIBED HEAT DISTRIBUTION OVER A PROBE BODY
APPAREIL ÉLECTROCHIRURGICAL DE PRODUCTION D'UNE DISTRIBUTION PRÉDÉTERMINÉE DE LA CHALEUR GRÂCE AU CORPS D'UNE SONDE

(30) Priorität: 10.07.2008 DE 102008032512; 01.09.2008 DE 102008045268
(43) Veröffentlichungstag der Anmeldung: 20.04.2011
(73) Patentinhaber: Erbe Elektromedizin GmbH, 72072 Tübingen (DE)
(72) Erfinder: GEISELHART, Franz, 72770 Reutlingen (DE); VOIGTLÄNDER, Matthias, 72810 Gomaringen (DE); KEGREISS, Horst, 72070 Tübingen (DE)
(74) Vertreter: Bohnenberger, Johannes
(86) Internationale Anmeldenummer: PCT/EP2009/004525
(87) Internationale Veröffentlichungsnummer: WO 2010/003547

(56) Entgegenhaltungen:
- WO-A-01/41664
- US-A- 4 946 460
- US-A1- 2005 010 201
- US-A1- 2007 149 959
- US-A1- 2008 154 258
- US-B1- 6 471 693

## Beschreibung

Die Erfindung betrifft ein elektrochirurgisches Gerät gemäß dem Anspruch 1, zur Erzeugung einer vorgegebenen Wärmeverteilung.

Es sind elektrochirurgische Geräte, insbesondere Sonden zur Devitalisierung von Gewebe bekannt (Ablationssonden), die einen Sondenkörper mit mindestens einer Elektrode zum Anlegen eines HF-Stroms und eine Kühleinrichtung umfassen. Der HF-Strom wird über einen HF-Generator (Hochfrequenzgenerator) erzeugt.

Bei der Hochfrequenzchirurgie wird Wechselstrom mit hoher Frequenz durch den menschlichen Körper geleitet, um Gewebe gezielt zu schädigen. Ein Anwendungsgebiet der Hochfrequenzchirurgie ist die Devitalisierung von Tumorgewebe. Die Hochfrequenzchirurgie macht sich den thermischen Effekt der Erwärmung zunutze, durch den eine Devitalisierung des Gewebes erzielt wird.

Man unterscheidet zwischen einer bipolaren und einer monopolaren Applikation des HF-Stroms. Bei einer monopolaren Anwendung umfasst das Instrument des elektrochirurgischen Geräts lediglich eine Elektrode, während eine zweite, Neutralelektrode unmittelbar an dem Patienten angebracht ist. Der Strom fließt umgekehrt proportional zu den Widerstandsverhältnissen im Gewebe von der Elektrode des Instruments zu der Neutralelektrode. In unmittelbarer Nähe der Elektrode des Instruments ist die Stromdichte derart hoch, dass der beschriebene thermische Effekt auftritt. Mit zunehmendem Abstand zu dieser Elektrode nimmt die Stromdichte quadratisch ab. Somit ist die devitalisierende Wirkung des HF-Stroms räumlich begrenzt.

Bei einer bipolaren Applikation umfasst das Instrument zwei Elektroden. Beispielsweise kann eine Sondenspitze als erste Elektrode ausgebildet sein, während ein proximalerer Abschnitt der Sonde als zweite Elektrode dient. Der HF-Strom bzw. die HF-Spannung liegt zwischen den beiden gegeneinander isolierten Elektroden an. Der Stromkreis wird über das dazwischen liegende Gewebe geschlossen. Es ergibt sich ein Stromverteilungsfeld, das sich in unmittelbarer Nachbarschaft zu der Sonde konzentriert.

Es ist offensichtlich, dass sich unabhängig von der Applikationsart des HF-Stroms eine hohe Felddichte in unmittelbarer Nachbarschaft zu dem Instrument einstellt. Diese hohe Stromdichte kann zu einer Karbonisation des umliegenden Gewebes führen. Diese Karbonisation ist zumindest bei der Devitalisierung von Tumoren unerwünscht, da eine entsprechende Schicht eine stark isolierende Wirkung hat und die Behandlung in tieferen Gewebebereichen hemmt. Des Weiteren kann der Körper entsprechend karbonisiertes Gewebe nicht ohne Weiteres abbauen.

Deshalb wird die Kühleinrichtung eingesetzt, um das unmittelbar benachbarte Gewebe zu kühlen und so eine Dehydration und/oder Karbonisation des anliegenden Gewebes zu vermeiden.

Bei der Devitalisierung von Tumorgeweben mit Hilfe einer Ablationssonde kann es vorkommen, dass benachbarte Strukturen (zum Beispiel Blutgefäße, Lymphbahnen, Organe) die Stromverteilung und damit die Wärmeverteilung nahe der Sonde beeinträchtigen. Bei monopolaren Sonden kann die Lage der Neutralelektrode relativ zu der Sonde oder dem Instrument zu einer unerwünschten Stromverteilung innerhalb des Gewebes führen. So ist es beispielsweise wünschenswert, bei der Behandlung von Tumoren ein möglichst gleichmäßiges, vorzugsweise sphärisches Wärmeverteilungsfeld bereit zu stellen, um den Tumor vollständig zu devitalisieren. WO 01/41664 ist der Erfindung ähnlich.

Ausgehend von diesem Stand der Technik, ist es Aufgabe der vorliegenden Erfindung, ein verbessertes elektrochirurgisches Gerät zur Devitalisierung von Gewebe bereit zu stellen. Insbesondere sollen herkömmliche elektrochirurgische Geräte derart verbessert werden, dass sie ein vorgegebenes Wärmeverteilungsfeld zur Devitalisierung des Gewebes generieren.

Diese Aufgabe wird erfindungsgemäß durch eine Vorrichtung nach Anspruch 1 gelöst.

Die Aufgabe wird durch ein elektrochirurgisches Gerät mit einer Sonde zur Devitalisierung von Gewebe gelöst, das umfasst:
- einen Sondenkörper,
- mindestens eine Elektrode zum Anlegen eines HF-Stroms an das Gewebe, mittels dessen eine Gewebeerwärmung bzw. ein Wärmeverteilungsfeld zur Devitalisierung des Gewebes generierbar ist,
- eine Kühleinrichtung zur Beeinflussung des Wärmeverteilungsfelds, wobei die Kühleinrichtung einen Verdunstungsbereich hat, der zum Kühlen mindestens eines Teils des Sondenkörpers über einen Zulauf mit einem Fluid versorgt wird,
- einen Ablauf zum Entsorgen des Fluids aus dem Verdunstungsbereich,
wobei der Verdunstungsbereich derart ausgebildet ist, dass eine Wärmeverteilung über den Söndenkörper durch das Variieren eines Zulaufdrucks in dem Zulauf und/oder eines Ablaufdrucks im Ablauf einstellbar ist.

Ein zentraler Gedanke der vorliegenden Erfindung besteht also darin, dass das mittels dem HF-Strom erzeugte Wärmeverteilungsfeld durch eine vorbestimmte Wärmeverteilung über den Sondenkörper beeinflusst wird. Somit lässt sich das Wärmeverteilungsfeld an die lokalen Gegebenheiten anpassen. Insbesondere können Strukturen, die eine sich von dem übrigen Gewebe unterscheidende Leitfähigkeit aufweisen und das Stromverteilungsfeld beeinflussen, berücksichtigt werden. Bei monopolaren Instrumenten ist es möglich, die Wärmeverteilung über den Sondenkörper derart einzustellen, dass variierende Abstände zwischen Abschnitten der Elektrode am Instrument und der Neutralelektrode das Wärmeverteilungsfeld nicht oder nur im geringen Maße beeinflussen.

Gemäß der vorliegenden Erfindung lässt sich die Wärmeverteilung auf dem Sondenkörper oder auf Teilen dessen durch das Variieren des Zulaufdrucks und/oder des Ablaufdrucks einstellen.

Das elektrochirurgische Gerät hat eine Steuereinrichtung zum Einstellen des Zulaufdrucks und/oder des Ablaufdrucks. Es ist denkbar, die Reglung der Druckverhältnisse oder des Zulaufdrucks oder des Ablaufdrucks in einem externen Gerät durchzuführen. Das erfindungsgemäße elektrochirurgische Gerät kann ein Ventil umfassen, um den Zulauf- oder den Ablaufdruck einzustellen.

Der Verdunstungsbereich umfasst mindestens ein Widerstands- oder Verwirbelungselement, das den Verdunstungsbereich in mindestens einen Distaldruckbereich und mindestens einen Proximaldruckbereich unterteilt und derart ausgebildet ist, dass sich die Druckverhältnisse zwischen dem Proximaldruckbereich und dem Distaldruckbereich mit zunehmendem Zulaufdruck verändern.

Zur Bereitstellung der Kühlleistung können entweder eine Kaltdampfanlage oder der Joule-Thomson-Effekt verwendet werden. Für beide Verfahren hängt die bereitgestellte Kühlleistung maßgeblich von den Druckverhältnissen innerhalb des Verdunstungsbereichs ab. Die Erfindung sieht vor, mindestens ein Verwirbelungselement oder Widerstandselement innerhalb des Verdunstungsbereichs anzuordnen, das den Verdunstungsbereich in mindestens zwei Druckzonen, nämlich den Proximaldruckbereich und den Distaldruckbereich unterteilt. Abhängig von dem Zulaufdruck oder dem Ablaufdruck können sich die Druckverhältnisse zwischen Proximaldruckbereich und Distaldruckbereich unterscheiden. Beispielsweise kann bei einem niedrigen Zulaufdruck, wobei nur wenig Fluid in den Verdunstungsbereich eingebracht wird, der Strömungswiderstand des Verwirbelungselements vernachlässigbar gering sein. Somit unterscheidet sich der Druck im Proximaldruckbereich nur geringfügig von dem im Distaldruckbereich. Erhöht man den Druck im Zulauf, so erhöht sich je nach Ausgestaltung des Widerstands oder Verwirbelungselments der Strömungswiderstand. Soweit der Zulauf in den Distaldruckbereich mündet, kann hier ein deutlich höherer Druck vorliegen als in dem Proximaldruckbereich. Bei gegebener Temperatur kann das für ein Kältemittel bedeuten, dass dieses im Distaldruckbereich nicht oder nur teilweise verdunstet, während im Proximaldruckbereich eine vollständige Verdunstung erfolgt. Eine entsprechende Veränderung des Druckverhältnisses in Abhängigkeit von dem Ablaufdruck oder einer Relation zwischen dem Zulaufdruck zu dem Ablaufdruck lässt sich ebenfalls gewährleisten.

Der Verdunstungsbereich kann eine Vielzahl von Widerstands- und/oder Verwirbelungselementen umfassen, die derart angeordnet und ausgebildet sind, dass sich mindestens ein Hauptdruckgefälle in dem Verdunstungsbereich in Abhängigkeit von dem Zulaufdruck und/oder Ablaufdruck, insbesondere entlang einer Längsachse der Sonde einstellt. Der Verdunstungsbereich kann also derart ausgebildet sein, dass sich entlang der Strömungsrichtung des Fluids ein definierter Strömungswiderstand ergibt. Z. B. können eine Vielzahl von Widerstandselementen darin angeordnet sein, die in Abhängigkeit von dem Zulaufdruck und/oder dem eingebrachten Fluidvolumen einen sich unterscheidenden Strömungswiderstand induzieren. Somit ist es möglich, das Hauptdruckgefälle in Abhängigkeit von dem Zulaufdruck und/oder Ablaufdruck zu variieren. Die maximale Kühlleistung wird jeweils dort auftreten, wo das Hauptdruckgefälle einen Siededruck oder Siededruckbereich unterschreiten. Somit ist es möglich, die maximale Kühlleistung je nach Bedarf innerhalb des Sondenkörpers zu positionieren.

Soweit es sich um eine längliche Sonde mit einer Längsachse handelt und der Verdunstungsbereich entlang dieser Längsachse ausgebildet ist, kann in Abhängigkeit von dem Zulaufdruck und/oder dem Ablaufdruck das Hauptdruckgefälle so eingestellt werden, dass das Kältemittel nahe dem proximalen oder distalen Ende der Sonde verdunstet. Eine kontinuierlich regelbare Position ist denkbar.

Mindestens eines der Verwirbelungselemente kann ein Expansionselement, insbesondere eine Expansionsdüse umfassen. Beispielsweise kann das Fluid über diese Expansionsdüse in den Verdunstungsbereich eingebracht werden. Je nach Einstellung des Zulaufdrucks und/oder des Ablaufdrucks tritt ein Siededruck unmittelbar hinter der Expansionsdüse auf. Durch eine Veränderung des Zulaufdrucks und/oder des Ablaufdrucks kann der Siededruck entlang der Strömungsrichtung verlagert werden. Beispielsweise kann durch eine Erhöhung des Zulaufdrucks der Strömungswiderstand an nachfolgenden Verwirbelungselementen derart erhöht werden, dass der Siededruck erst nach diesen Verwirbelungselementen derart stark abfällt, dass es zu einer Verdunstung kommt.

Das elektrochirurgische Gerät umfasst eine Steuerungseinrichtung, die den das Gewebe erwärmenden HF-Strom und den eine Kühlung der Sonde bestimmenden Zulaufdruck und/oder Ablaufdruck derart einstellt, dass sich bei der Applikation des HF-Stroms ein vorgegebenes, insbesondere im Wesentlichen sphärisches Wärmeverteilungsfeld im Gewebe ergibt. Beispielsweise kann es zur Behandlung von Tumoren hilfreich sein, wenn sich ein Wärmeverteilungsfeld ergibt, das symmetrisch, insbesondere sphärisch ausgebildet ist. Das Wärmeverteilungsfeld kann derart definiert sein, dass es sich hierbei um den dreidimensionalen Bereich nahe dem Sondenkörper handelt, der durch den HF-Strom erhitzt wird. Insbesondere kann das besagte Wärmeverteilungsfeld derart definiert werden, dass das Gewebe innerhalb dieses Bereichs derart stark erhitzt wird, dass es zu einer Devitalisierung kommt.

Bei der Bereitstellung der Wärmeverteilung besteht also ein zentraler Gedanke der vorliegenden Erfindung darin, die Position der maximalen Kühlleistung über ein Einstellen des Hauptdruckgefälles vorzunehmen. Je nach der Ausgestaltung des Hauptdruckgefälles findet der Siedevorgang des Kältemittels an einer anderen Position im Verdunstungsbereich statt.

Nachfolgend wird die Erfindung anhand einiger Ausführungsbeispiele beschrieben, die mittels Abbildungen näher erläutert werden. Hierbei zeigen:
- Fig. 1 wesentliche Bestandteile eines elektrochirurgischen Geräts;
- Fig. 2 eine bipolare Ablationssonde mit Wärmeverteilungsfeld bei stärkerer Kühlung im Proximalbereich;
- Fig. 3 eine bipolare Ablationssonde mit Wärmeverteilungsfeld bei stärkerer Kühlung im Distalbereich;
- Fig. 4 eine monopolare Ablationssonde mit Wärmeverteilungsfeld bei stärkerer Kühlung im Distalbereich;
- Fig. 5 einen Querschnitt durch eine Ablationssonde mit Widerstandselementen in Lamellenanordnung;
- Fig. 6 einen Querschnitt durch eine Ablationssonde mit spiralförmigen Widerstandselementen; und
- Fig. 7 einen Querschnitt durch eine Ablationssonde mit dreieckigen Widerstandselementen.

In der nachfolgenden Beschreibung werden für gleiche und gleich wirkende Teile dieselben Bezugsziffern verwendet.

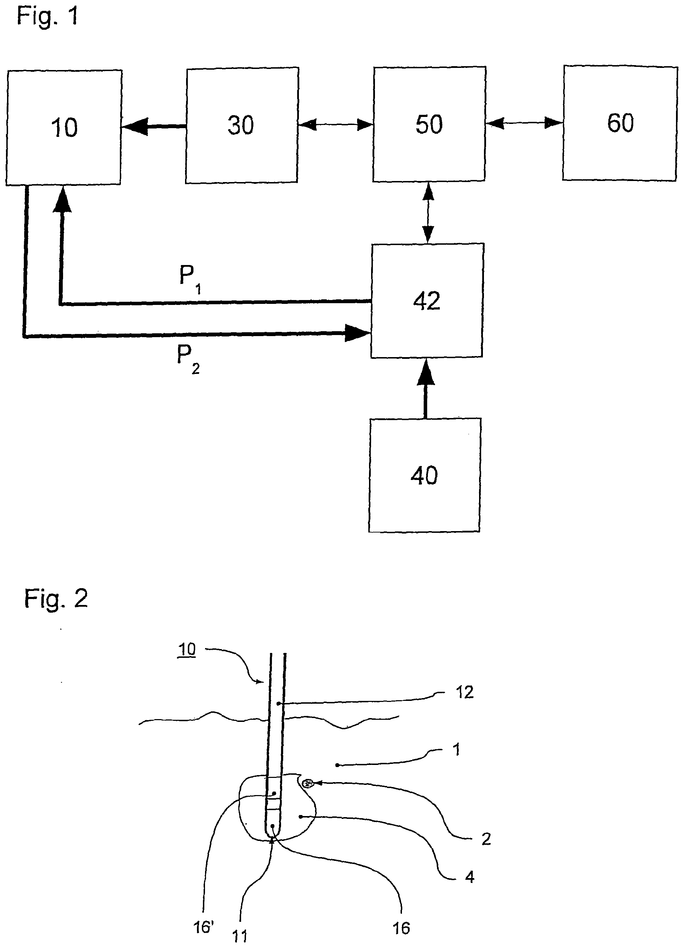

Die Fig. 1 zeigt die wesentlichen Komponenten eines erfindungsgemäßen elektrochirurgischen Geräts. Eine Ablationssonde 10 wird üblicherweise aus einer Fluidquelle 40 mit Fluid versorgt. Zur Einstellung eines vorgegebenen Zulaufdrucks P1 und/oder Ablaufdrucks P2 steht die Fluidquelle 40 in fluider Verbindung mit einer Fluidregeleinrichtung 42, die eine Vielzahl von Ventilen und Messfühlern umfassen kann.

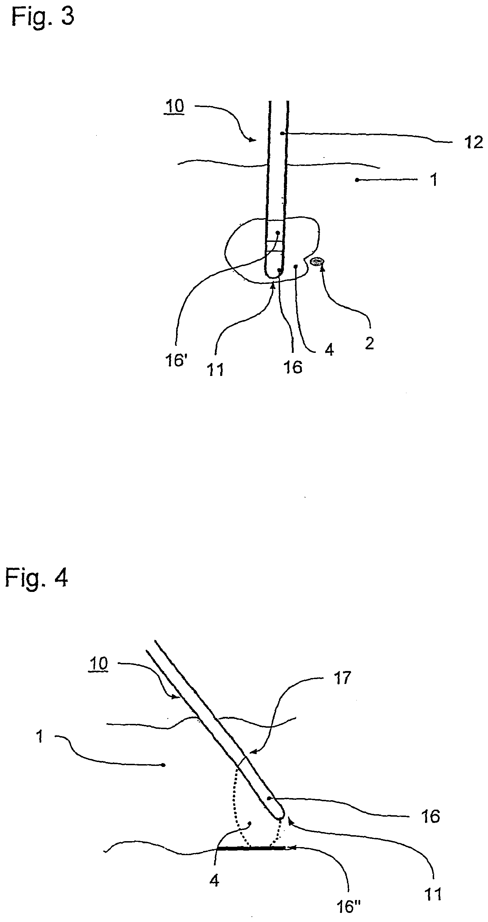

Ein HF-Generator 30 steht in elektrischer Verbindung mit der Ablationssonde 10 und stellt einen HF-Strom bereit, der über entsprechende Elektroden 16, 16' (vergleiche Fig. 2) oder 16" (vergleiche Fig. 4) an einem zu behandelnden Gewebe 1 angelegt wird. Das elektrochirurgische Gerät umfasst des Weiteren eine Steuerung 50, die über entsprechende Stellsignale den HF-Generator 30 und die Fluidregeleinrichtung 42 steuert. Die Steuerung 50 empfängt Signale von einer Bedieneinheit 60, über die der behandelnde Arzt Einstellungen an dem elektrochirurgischen Gerät vornehmen kann. Beispielsweise kann über die Bedieneinheit 60 der Koagulationsvorgang gestartet werden.

Fig. 2 zeigt ein erstes Ausführungsbeispiel einer erfindungsgemäßen Ablationssonde 10. Hierbei handelt es sich um eine bipolare Ablationssonde 10, die eine erste Elektrode 16 und eine zweite Elektrode 16' aufweist. Beide Elektroden 16, 16' befinden sich in einem Distalbereich eines Sondenkörpers 12, nahe einer Sondenspitze 11. Die beiden Elektroden 16, 16' sind gegeneinander elektrisch isoliert.

Sobald ein HF-Strom an den Elektroden 16, 16' angelegt wird, entsteht in dem die Ablationssonde 10 kontaktierenden Gewebe 1 ein Stromverteilungsfeld. Soweit ein homogenes Gewebe 1 vorliegt, ist dieses Stromverteilungsfeld im Wesentlichen sphärisch ausgebildet, wobei die Stromdichte mit zunehmender Distanz von dem Sondenkörper 12 abnimmt. Der HF-Strom verrichtet an dem Gewebe 1 Arbeit, die in Form von Wärmeenergie freigesetzt wird. In Abhängigkeit von der Ausgestaltung des Gewebes 1 ergibt sich ein Wärmeverteilungsfeld. Innerhalb des Wärmeverteilungsfelds gibt es einen Bereich in dem die Temperaturerhöhung derart hoch ist, dass das Gewebe devitalisiert wird. Dieser Bereich wird als Koagulationszone 4 definiert.

Unregelmäßige Strukturen in dem Gewebe 1, beispielsweise Blutgefäße, Lymphbahnen oder Organe können das Stromverteilungsfeld derart beeinflussen, dass ein unsymmetrisches Wärmeverteilungsfeld entsteht. Häufig ist ein unsymmetrisches Wärmeverteilungsfeld und somit eine unsymmetrische Koagulationszone 4 unerwünscht.

In dem in Fig. 2 gezeigten Anwendungsbeispiel befindet sich ein Blutgefäß 2 in mittelbarer Nachbarschaft zu der Ablationssonde 10. Bei herkömmlichen Ablationssonden 10 würde sich hieraus eine Koagulationszone 4 ergeben, die eine starke Asymmetrie im Bereich des Blutgefäßes 2 aufweist.

Die erfindungsgemäße Ablationssonde 10 kann diesem Effekt jedoch entgegen wirken. Hierfür wird die zweite Elektrode 16', die sich proximal hinter der ersten Elektrode 16 befindet, stärker gekühlt. Es ergibt sich die in Fig. 2 gezeigte annähernd symmetrische Koagulationszone 4.

Fig. 3 zeigt ein weiteres Anwendungsbeispiel der erfindungsgemäßen Ablationssonde 10. Hier befindet sich ein Blutgefäß 2 nahe der ersten Elektrode 16 und somit in mittelbarer Nachbarschaft zur Sondenspitze 11. Einer daraus resultierenden Asymmetrie der Koagulationszone 4 kann durch ein stärkeres Kühlen des Distalbereichs der Ablationssonde 10, also der ersten Elektrode 16 entgegen gewirkt werden. Betrachtet man also die Wärmeverteilung auf dem Sondenkörper 12, so ergibt sich eine maximale Kühlleistung (minimale Temperatur) im Bereich der Sondenspitze 11, die in proximaler Richtung abnimmt. Dementsprechend ergibt sich bei einer distalen Einbringung des Fluids in dem Verdunstungsbereich ein abnehmendes Hauptdruckgefälle in proximale Richtung.

Die Fig. 4 zeigt ein weiteres Ausführungsbeispiel der erfindungsgemäßen Ablationssonde 10. Es handelt sich hierbei um eine monopolare Sonde. Die Ablationssonde 10 umfasst also nur eine Elektrode 16. Die zweite Elektrode zur Applikation des HF-Stroms ist eine Neutralelektrode 16", die großflächig an einer Gewebeoberfläche des Gewebes 1 angebracht ist. In Abhängigkeit von der Position und der Ausrichtung der Ablationssonde 10 zu der großflächigen Neutralelektrode 16" ergibt sich ein Stromverteilungsfeld, das von den Distanzen zwischen den Elektroden 16, 16" abhängt. Im in Fig. 4 gezeigten Anwendungsbeispiel ist die Stromdichte im Gewebe 1 nahe der Sondenspitze 11 wesentlich höher als in Bereichen, die zwischen einem proximalen Ende der Elektrode 16 und der Neutralelektrode 16" liegen. Somit kann es nahe der Sondenspitze 11 zu einer unerwünschten Karbonisierung des Gewebes 1 kommen. Um diesen Effekt zu vermeiden, kühlt die vorliegende Ablationssonde 10 den Sondenkörper 12 stärker im Bereich nahe der Sondenspitze 11. Insbesondere wird der Distalbereich der Elektrode 16 stärker gekühlt als ein Proximalbereich.

Es sind Positionen und Ausrichtungen der monopolaren Ablationssonde 10 denkbar, in denen der Proximalbereich der Elektrode 16 stärker gekühlt werden muss, um ein möglichst gleichmäßig abnehmendes Wärmeverteilungsfeld zu erzeugen.

Für die vorliegende Erfindung ist es wichtig, die Wärmeverteilung am Sondenkörper 12 der Ablationssonde 10 einstellen zu können. Insbesondere kann die erfindungsgemäße Ablationssonde 10 eine maximale Kühlleistung entlang ihrer Längsachse wandern lassen. Somit ist es möglich, eine maximale Kältezone an der Spitze 11 der Ablationssonde 10 einzustellen. Alternativ kann die Kältezone nahe dem proximalen Ende 17 der Elektrode 16 positioniert werden.

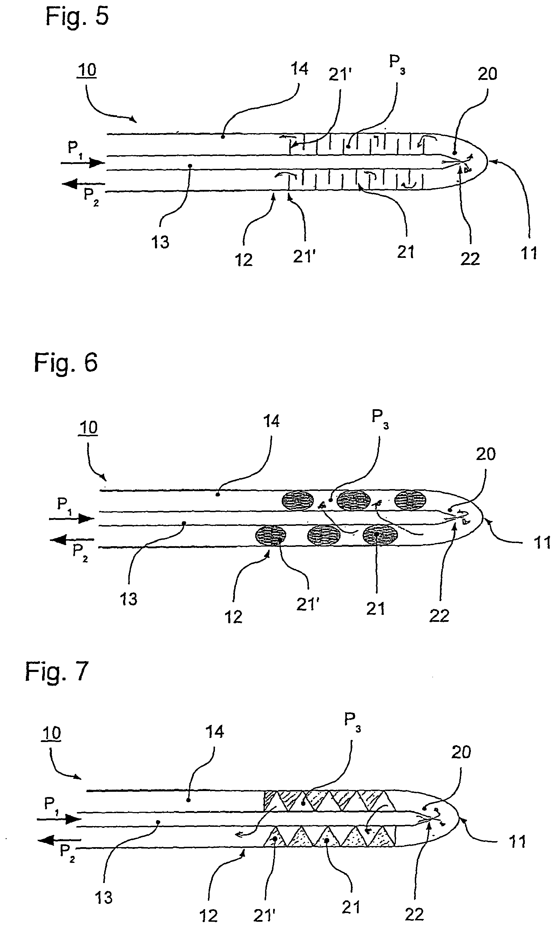

Die in den Fig. 5 bis 7 gezeigten Ausführungsbeispiele einer Ablationssonde 10 ermöglichen eine Einstellung bzw. Positionierung des Kältezentrums über den Zulaufdruck P1. Diese Ablationssonden 10 umfassen einen Verdunstungsbereich 20, in dem ein Kältemittel verdunstet und der Ablationssonde 10, insbesondere dem Sondenkörper 12 Wärmeenergie entzieht. Der Verdunstungsbereich 20 erstreckt sich von der Sondenspitze 11 in proximaler Richtung entlang der Längsachse der Ablationssonde 10.

Das Kältemittel wird über einen Zulauf 13 nahe der Sondenspitze 11 in den Verdunstungsbereich 20 eingebracht. Da der Siedepunkt kältemittelspezifisch von dem vorliegenden Druck und der Temperatur abhängt, verdunstet das Kältemittel bei gegebener Temperatur nur dann, wenn in dem Verdunstungsbereich 20 nahe der Sondenspitze 11 ein Druck P3 vorliegt, der unterhalb des Siededrucks liegt.

Der Verdunstungsbereich 20 umfasst eine Vielzahl von Verwirbelungselementen 21, 21', die gemäß Fig. 5 derart abwechselnd angeordnet sind, dass das Kältemittel auf seinem Rückweg zu dem Ablauf 14 einzelne Lamellen, die die Verwirbelungselemente 21, 21' bilden, passieren muss. Es entstehen Turbulenzen. Diese Turbulenzen verbessern unter anderem den Wärmeübergang zwischen dem Kältemittel und der Ablationssonde 10, zum anderen bewirken sie einen Strömungswiderstand, der in Abhängigkeit von dem Zulaufdruck P1 zunimmt.

Somit ergibt sich bei einem geringen Zulaufdruck P1 knapp unterhalb des Siededrucks nur ein geringer Strömungswiderstand. Das Kältemittel verdunstet im distalen Bereich des Verdunstungsbereichs 20 nahe einer Expansionsdüse 22. Das Hauptdruckgefälle unterschreitet also bereits im distalen Bereich des Verdunstungsbereichs den Siededruck. Mit zunehmendem Zulaufdruck P1 steigt der Strömungswiderstand. Hierdurch ergibt sich ein Rückstau, der in distaler Richtung des Verdunstungsbereichs 20 zunimmt. Die Verwirbelungselemente 21, 21' sind derart angeordnet und ausgebildet, dass Druck P3 in Flussrichtung abnimmt. Somit ist eine Einstellung des
Zulaufdrucks P1 denkbar, bei der eine Verdunstung des Kältemittels erst nach dem letzten Verwirbelungselement 21' auftritt. Der Druck P3 des Hauptdruckgefälles bleibt also bis zu diesem letzten Verwirbelungselement 21' oberhalb des Siededrucks. Durch eine Variation des Zulaufdrucks P1 lässt sich das Hauptdruckgefälle des Drucks P3 im Verdunstungsbereich 20 derart einstellen, dass die Zone der Verdunstung, also der maximalen Kühlung beliebig positioniert werden kann.

Fig. 6 und 7 zeigen weitere Ausführungsbeispiele der Verwirbelungselemente 21, 21'. In Fig. 6 sind die Verwirbelungselemente 21, 21' als eine Spirale ausgebildet, die sich wendelförmig um den Zulauf 13 schlängelt.

In Fig. 7 sind die Verwirbelungselemente 21, 21' als Zacken ausgebildet, die in den Verdunstungsbereich ragen.

### Bezugszeichenliste

- 1: Gewebe
- 2: Blutgefäß
- 4: Koagulationszone
- 10: Ablationssonde
- 11: Sondenspitze
- 12: Sondenkörper
- 13: Zulauf
- 14: Ablauf
- 16, 16': Elektroden
- 16": Neutralelektrode
- 17: proximales Ende der Elektrode 16
- 20: Verdunstungsbereich
- 21, 21': Verwirbelungselement
- 22: Expansionsdüse
- 30: HF-Generator
- 40: Fluidquelle
- 42: Fluidregeleinrichtung
- 50: Steuerung
- 60: Bedieneinheit
- P1: Zulaufdruck
- P2: Ablaufdruck
- P3: Druck im Verdunstungsbereich 20

## Patentansprüche

1. Elektrochirurgisches Gerät zur Devitalisierung von Gewebe (1), umfassend:
- einen Sondenkörper (12),
- mindestens eine Elektrode (16, 16', 16") zum Anlegen eines HF-Stroms an das Gewebe (1), mittels dessen eine Gewebeerwärmung bzw. ein Wärmeverteilungsfeld zur Devitalisierung des Gewebes generierbar ist,
- eine Kühleinrichtung zur Beeinflussung des Wärmeverteilungsfelds, wobei die Kühleinrichtung einen Verdunstungsbereich (20) hat, der zum Kühlen mindestens eines Teils des Sondenkörpers (12) über einen Zulauf (13) mit einem Fluid versorgt wird,
- einen Ablauf (14) zum Entsorgen des Fluids aus dem Verdunstungsbereich (20),
- eine Steuereinrichtung zum Einstellen eines Zulaufdrucks (P1),
wobei der Verdunstungsbereich (20) mindestens ein Widerstands- oder Verwirbelungselement (21, 21') umfasst, das den Verdunstungsbereich (20) in mindestens einen Distaldruckbereich und mindestens einen Proximaldruckbereich unterteilt, wobei der Zulauf (13) in den Distaldruckbereich mündet,
wobei
der Verdunstungsbereich (20) derart ausgebildet ist, dass eine Wärmeverteilung über den Sondenkörper (12) durch das Variieren des Zulaufdrucks (P1) in dem Zulauf (13) und/oder eines Ablaufdrucks (P2) im Ablauf (14) einstellbar ist,
wobei
die Steuerungseinrichtung, die den das Gewebe erwärmenden HF-Strom und den eine Kühlung der Sonde bestimmenden Zulaufdruck (P1) und/oder Ablaufdruck (P2) derart einstellt, dass sich bei der Applikation des HF-Stroms ein vorgegebenes Wärmeverteilungsfeld im Gewebe ergibt,
wobei der Verdunstungsbereich (20) derart ausgebildet ist, dass sich das Druckverhältnis zwischen Proximaldruckbereich und Distaldruckbereich mit zunehmendem Zulaufdruck (P1) verändert, "**dadurch gekennzeichnet, dass** die Steuereinrichtung dazu ausgebildet ist, den Zulaufdruck (P1) zu erhöhen, um eine stärkere Kühlung im Proximaldruckbereich zu erreichen.

2. Elektrochirurgisches Gerät nach Anspruch 1,
**gekennzeichnet durch**
eine Einstellvorrichtung (41) zum Einstellen des Zulaufdrucks (P1) und/oder des Ablaufdrucks (P2).

3. Elektrochirurgisches Gerät nach einem der vorhergehenden Ansprüche,
**dadurch gekennzeichnet, dass**
der Verdunstungsbereich (20) eine Vielzahl von Widerstands- und/oder Verwirbelungselementen (21, 21') umfasst, die derart angeordnet und ausgebildet sind, dass sich ein Hauptdruckgefälle in dem Verdunstungsbereich (20) in Abhängigkeit von dem Zulaufdruck (P1) und/oder Ablaufdruck (P2), insbesondere entlang einer Längsachse der Sonde (10) einstellt.

4. Elektrochirurgisches Gerät nach einem der vorhergehenden Ansprüche,
**dadurch gekennzeichnet, dass**
mindestens eines der Verwirbelungselemente (21, 21') ein Expansionselement, insbesondere eine Expansionsdüse (22) umfasst.

## Claims

1. Electrosurgical appliance for devitalization of tissue (1), comprising:
- a probe body (12),
- at least one electrode (16, 16', 16") for applying an HF current to the tissue (1), by means of which HF current the tissue can be heated or a heat distribution field for tissue devitalization can be generated,
- a cooling device for influencing the heat distribution field, wherein the cooling device has an evaporation region (20) which, in order to cool at least part of the probe body (12), is supplied with a fluid via an inlet (13),
- an outlet (14) for removing the fluid from the evaporation region (20),
- a control device for adjusting an inlet pressure (P1),
wherein the evaporation region (20) comprises at least one resistance element or swirl element (21, 21') which divides the evaporation region (20) into at least one distal pressure region and at least one proximal pressure region, wherein the inlet (13) opens into the distal pressure region,
wherein the evaporation region (20) is configured in such a way that a heat distribution is adjustable via the probe body (12) by varying the inlet pressure (P1) in the inlet (13) and/or an outlet pressure (P2) in the outlet (14),
wherein the control device adjusts the HF current heating the tissue, and the inlet pressure (P1) and/or outlet pressure (P2) determining the cooling of the probe, in such a way that, when the HF current is applied, a predefined heat distribution field is produced in the tissue,
wherein the evaporation region (20) is configured in such a way that the pressure ratio between proximal pressure region and distal pressure region changes as the inlet pressure (P1) increases, **characterized in that** the control device is configured to increase the inlet pressure (P1) in order to achieve stronger cooling in the proximal pressure region.

2. Electrosurgical appliance according to Claim 1, **characterized by** an adjusting device (41) for adjusting the inlet pressure (P1) and/or the outlet pressure (P2).

3. Electrosurgical appliance according to one of the preceding claims, **characterized in that** the evaporation region (20) has a multiplicity of resistance and/or swirl elements (21, 21'), which are arranged and configured in such a way that a main pressure gradient is established in the evaporation region (20), in particular along a longitudinal axis of the probe (10), depending on the inlet pressure (P1) and/or outlet pressure (P2)

4. Electrosurgical appliance according to one of the preceding claims, **characterized in that** at least one of the swirl elements (21, 21') comprises an expansion element, in particular an expansion nozzle (22) .

## Revendications

1. Appareil électrochirurgical destiné à la dévitalisation de tissu (1), comprenant:
- un corps de sonde (12),
- au moins une électrode (16, 16', 16") pour appliquer un courant HF au tissu (1), au moyen duquel il est possible de produire un échauffement du tissu ou un champ de distribution de chaleur pour la dévitalisation du tissu,
- un dispositif de refroidissement pour influencer le champ de distribution de chaleur, dans lequel le dispositif de refroidissement comporte une zone d'évaporation (20), qui est alimentée en fluide par une arrivée (13) pour le refroidissement d'au moins une partie du corps de sonde (12),
- une évacuation (14) pour évacuer le fluide hors de la zone d'évaporation (20),
- un dispositif de commande pour le réglage de la pression d'arrivée (P1),
dans lequel la zone d'évaporation (20) comprend au moins un élément de résistance ou de turbulence (21, 21'), qui divise la zone d'évaporation (20) en au moins une zone de pression distale et au moins une zone de pression proximale, dans lequel l'arrivée (13) débouche dans la zone de pression distale,
dans lequel la zone d'évaporation (20) est configurée de telle manière qu'une distribution de chaleur sur le corps de sonde (12) puisse être réglée par la variation de la pression d'arrivée (P1) dans l'arrivée (13) et/ou d'une pression d'évacuation (P2) dans l'évacuation (14),
dans lequel le dispositif de commande, qui règle le courant HF échauffant le tissu et la pression d'arrivée (P1) et/ou une pression d'évacuation (P2) déterminant un refroidissement de la sonde de telle manière que lors de l'application du courant HF il s'établisse un champ de distribution de chaleur prédéterminé dans le tissu, dans lequel la zone d'évaporation (20) est configurée de telle manière que le rapport de pression entre la zone de pression proximale et la zone de pression distale varie avec l'augmentation de la pression d'arrivée (P1), **caractérisé en ce que** le dispositif de commande est configuré pour augmenter la pression d'arrivée (P1) afin d'atteindre un refroidissement plus intense dans la zone de pression proximale.

2. Appareil électrochirurgical selon la revendication 1, **caractérisé par** un dispositif de réglage (41) pour le réglage de la pression d'arrivée (P1) et/ou de la pression d'évacuation (P2).

3. Appareil électrochirurgical selon l'une quelconque des revendications précédentes, **caractérisé en ce que** la zone d'évaporation (20) comprend une multiplicité d'éléments de résistance et/ou de turbulence (21, 21'), qui sont disposés et configurés de telle manière qu'il s'établisse un gradient de pression principal dans la zone d'évaporation (20) en fonction de la pression d'arrivée (P1) et/ou de la pression d'évacuation (P2), en particulier le long d'un axe longitudinal de la sonde (10).

4. Appareil électrochirurgical selon l'une quelconque des revendications précédentes, **caractérisé en ce qu'**au moins un des éléments de turbulence (21, 21') comprend un élément d'expansion, en particulier une buse d'expansion (22).
